(19) Europäisches Patentamt

European Patent Office

Office européen des brevets

(11) Veröffentlichungsnummer: **0 267 404 B1**

(12) **EUROPÄISCHE PATENTSCHRIFT**

(45) Veröffentlichungstag der Patentschrift: **10.03.93**

(21) Anmeldenummer: **87114168.5**

(22) Anmeldetag: **29.09.87**

(51) Int. Cl.⁵: **A61K 31/495**

Die Akte enthält technische Angaben, die nach dem Eingang der Anmeldung eingereicht wurden und die nicht in dieser Patentschrift enthalten sind.

(54) **Dermale Behandlung von Wurmerkrankungen der Katze mit Praziquantel.**

(30) Priorität: **11.10.86 DE 3634755**

(43) Veröffentlichungstag der Anmeldung:
**18.05.88 Patentblatt 88/20**

(45) Bekanntmachung des Hinweises auf die
Patenterteilung:
**10.03.93 Patentblatt 93/10**

(84) Benannte Vertragsstaaten:
**BE CH DE ES FR GB IT LI NL SE**

(56) Entgegenhaltungen:
**DE-A- 3 619 030**

**Z. PARASITENK., Band 52, 1977, Springer-
Verlag (DE); R.GÖNNERT et al., Seiten
129-150***

**Z. PARASITENK., Band 52, 1977, Springer-
Verlag (DE); H.THOMAS et al., Seiten 117-127***

**RESEARCH IN VETERINARY SCIENCE, Band
24, 1978; H.THOMAS et al., Seiten 20-25***

(73) Patentinhaber: **BAYER AG**

**W-5090 Leverkusen 1 Bayerwerk(DE)**

(72) Erfinder: **Andrews, Peter, Dr.
Gellertweg 2
W-5600 Wuppertal 1(DE)**
Erfinder: **Voege, Herbert, Dr.
Martin Buber-Str. 41
W-5090 Leverkusen 3(DE)**

**Beschreibung**

Die vorliegende Erfindung betrifft die dermale Anwendung von Praziquantel zur Bekämpfung von Wurmerkrankungen der Katze sowie dafür geeignete Mittel.

Es ist bekannt, Praziquantel zur Bekämpfung von Wurmerkrankungen von Tieren z.B. Hund und Katze einzusetzen (US-A-4113867; P. Andrews et.al. Medicinal Research Rev. Vol 3 2 147 - 200 (1983)). Die Behandlung erfolgt bei Hund und Katze durch orale oder parenterale Verabreichung des Wirkstoffs in Dosen von ca. 5 mg/kg Körpergewicht. Bevorzugt ist die orale Behandlung, bei der der Wirkstoff direkt in den Lebensraum des Parasiten verabreicht wird. Eine sehr gute Wirksamkeit in niedrigen Dosen kann daher bei dieser Art der Verabreichung erwartet werden.

Eine deutlich schlechtere Wirksamkeit muß erwartet werden, wenn der Wirkstoff dermal verabreicht wird. Dies ist auch allgemein bekannt (Herlich et. al Veterinary Med. 56, p 219 - 221 (1961); Hotson et. al. Australian Vet. J. 39; p 108 - 115 (1963)). Untersuchungen mit Praziquantel bei Ratten zeigen, daß sowohl bei dermaler als auch bei subcutaner Verabreichung von Praziquantel höhere Wirkstoffdosen erforderlich sind als bei oraler Verabreichung (H. Thomas et.al. Z. Parasitenkd. 52; 117 - 127 (1977).

Bei Untersuchungen gegen Schistosoma mansoni in Mäusen zeigte sich, daß bei oraler Verabreichung des Wirkstoffs nur ein Zehntel der Menge die dermal verabreicht wurde erforderlich war, um die gleiche Wirkung zu erzielen (R. Gönnert et.al. Z. Parasitenkd. 52; p. 129-150 (1977)).

Bei Untersuchungen gegen Taenia hydatigena in Hunden wurde ebenfalls gefunden, daß die dermale Anwendungsform nur unbefriedigende Ergebnisse zeigte. Bei oraler Anwendung wird eine Dosis von 1 mg/kg empfohlen. Die dermale Anwendung einer Lösung der Wirkstoffe in Isopropanol bei einer Dosis von 5 mg/kg zeigte nur bei einem von 2 Hunden Wirkung (H.Thomas et.al. Research Vet. Sci. 24; p. 20-25 (1978)).

Es war daher zu erwarten, daß bei dermaler Anwendung von Praziquantel höhere Dosen (ca. 10-fach) erforderlich sind als bei oraler Anwendung.

Dermale Mittel müssen aber neben der Wirksamkeit auch bestimmte Erwartungen des Verbrauchers erfüllen. Auch nach der Anwendung darf das Fell der Tiere nicht naß aussehen oder sich feucht anfühlen. Außerdem muß die Menge des zur Anwendung kommenden Lösungsmittels so gering wie möglich gehalten werden, um schädliche Wirkungen des Lösungsmittels auf das Tier zu vermeiden. Der Lösungsmittelaufwand soll daher 0,1 ml/kg Katze-Lebendgewicht nicht überschreiten. Es war kaum möglich Lösungsmittel zu finden, die für die Katze unschädlich sind und die für die Wirksamkeit erforderliche Menge Praziquantel lösen. Dies war mit ein Grund dafür weshalb es bis jetzt keine dermal anwendbaren Mittel, die Praziquantel enthalten, für die Katze gibt.

Es wurden neue Mittel zur dermalen Bekämpfung von Wurmerkrankungen gefunden, die Praziquantel in Konzentrationen von 0,1 - 20 Gewichtsprozent neben N-Methylpyrrolidon und/oder Isopropylmyristat sowie gegebenenfalls weiteren Lösungsmitteln und Hilfsstoffen enthalten.

Es wurde nun gefunden, daß man Praziquantel zur dermalen Bekämpfung von Wurmerkrankungen der Katze in Aufwandmengen von 0,1 - 5 mg pro kg Körpergewicht in Mitteln einsetzen kann, die N-Methylpyrrolidon und/oder Isopropylmyristat enthalten. Es wurde gefunden, daß von diesen Mitteln 0,01 - 0,5 ml/kg Katze-Lebendgewicht zur Bekämpfung von Wurmerkrankungen der Katze verabreicht werden müssen.

Es bar überraschend, daß mit dieser Behandlung Wurmerkrankungen der Katze erfolgreich bekämpft werden können. Erstaunlicherweise reicht bei der dermalen Behandlung der Katze, im Gegensatz zu den Angaben aus dem Stand der Technik, die Menge Praziquantel aus, die auch für die orale Anwendung erforderlich ist. Damit können, trotz der Forderung nach Anwendung geringer Lösungsmittelmengen ausreichende Wirkstoffmengen auf die Katze aufgebracht werden.

Der Vorteil der dermalen Anwendung liegt auf der Hand. Das Tier braucht vor der Behandlung nicht fixiert zu werden wie dies bei der Verabreichung als Injektion notwendig ist. Es braucht auch nicht mit medikiertem Futter behandelt zu werden, wobei die Gefahr der Futterverweigerung besteht. Es genügt ein einfaches Auftropfen der Formulierung auf das Tier, z. B. zwischen die Schulterblätter um zu vermeiden, daß das Tier das Mittel vom Fell ableckt.

Infolge der geringen Wirkstoffmenge die zur Anwendung kommt, können Lösungsmittel ausgewählt werden, in denen ausreichende Mengen Wirkstoff gelöst sind und die weder die Haut des Tieres reizen noch das Tier dann schädigen wenn es sich das Mittel vom Fell leckt.

Praziquantel, common name für 2-(Cyclohexyl-carbonyl)-1,2,3,6,7,11-b-hexahydro-4 H-pyrazino[2,1-a]-isochinolin-4-on ist bekannt.

Wie bereits erwähnt wird Praziquantel bei dem erfindungsgemäßen Verfahren in Aufwandmengen von 0,1 -5 mg, bevorzugt von 1 - 5 mg besonders bevorzugt von ca. 1 mg pro kg Körpergewicht eingesetzt.

EP 0 267 404 B1

Die erfindungsgemäßen Mittel enthalten 0,1 - 20, bevorzugt 1 - 5, besonders bevorzugt ca. 1, Gewichtsprozent Praziquantel neben Lösungsmitteln und gegebenenfalls weiteren Hilfsstoffen.

Die erfindungsgemäßen Mittel werden in Mengen von 0,01 - 0,5 ml, bevorzugt 0,1 - 0,5 ml, besonders bevorzugt ca. 0,1 ml, pro kg Lebendgewicht der Katze eingesetzt.

Die erfindungsgemäßen Mittel werden auf begrenzte Bereiche des Fells der Katze aufgespritzt, aufgetropft, aufgeträufelt oder aufgesprüht. Bevorzugt werden Stellen der Tiere behandelt, von denen das Mittel nicht ohne weiteres abgeleckt werden kann.

Die erfindungegemäßen Mittel werden hergestellt, indem man den Wirkstoff in N-Methylpyrrolidon und/oder Isopropylmyristat löst und gegebenenfalls weitere geeignete hautverträgliche Lösungsmittel oder Lösungsmittelgemische zugibt. Gegebenenfalls werden weitere Hilfsstoffe wie Farbstoffe, resorptionsfördernde Stoffe, Antioxidantien, Lichtschutzmittel oder Haftmittel zugefügt.

Als gut geeignete Lösungsmittel kommen für die Herstellung der erfindungsgemäßen Mittel in Betracht: Alkanole, wie Äthylalkohol, Isopropylalkohole, n-Butylalkohol, Amylalkohol.

Glykole, wie Propylenglykol, Glycerin, 1,3-Butylenglykol, Polyethylenglycole, Polypropylenglycole.

Aromatische Alkohole wie Benzylalkohol, Phenylethanol, Phenoxyethanol.

Dreiwertige Alkohole wie Glycerin.

Carbonsäureester, wie z.B. Äthylacetat, Benzylbenzoat, Butylacetat, Milchsäureethylester.

Aromatische und/oder aliphatische Kohlenwasserstoffe.

Öle, wie z.B. Baumwollsaatöl, Erdnußöl, Maiskernöl, Olivenöl, Ricinusöl, Sesamöl oder synthetische Analoge dieser Öle.

Wasser.

Ketone, wie z.B. Aceton und Methylethylketon.

Ether wie Akkylenglycolalkylether, Dipropylenglycolmonomethylether, Diethylenglycolmonobutylether.

Ferner Dimethylformamid, Dimethylacetamid, N-Methylpyrrolidon und 2-Dimethyl-4-oxymethylen-1,3-dioxolan.

Die erfindungsgemäßen Mittel können auch Emulgatoren und Netzmittel enthalten, wie z.B.

anionaktive Tenside, wie z.B. Na-Laurylsulfat, Fettalkoholäthersulfate, Mono/Dialkylpolyglykolätherorthophosphorsäureester-Monoäthanolaminsalz;

kationaktive Tenside wie z.B. Cetyltrimethylammoniumchlorid;

ampholytische Tenside wie z.B. Di-Na-N-lauryl-$\beta$-iminodipropionat oder Lecithin;

nicht ionogene Tenside wie z.B. polyoxäthyliertes Rizinusöl, polyoxyäthyliertes Sorbitan-Monoleat, Sorbitan Monostearat, Äthylalkohol, Glycerinmonostearat, Polyoxyätherylenstearat, Alkylphenolpolyglykoläther.

Außerdem können die erfindungsgemäßen Mittel resorptionsfördernde Stoffe enthalten wie z.B. DMSO. Außerdem können sie spreitende Öle enthalten. Zu den spreitenden Ölen zählen u.a.:

Silikonöle verschiedener Viskosität,

Fettsäureester wie Aethylstearat, Di-n-butyl-adipat, Laurinsäurehexylester, Dipropylen-glykolpelargonat, Ester einer verzweigten Fettsäure mittlerer Kettenlänge mit gesättigten Fettalkoholen $C_6$ - $C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Aethyloleat, Milchsäureaethylester, Dibutylphthalat, Adipinsäurediisopropylester, Triglyceride wie Capryl/Caprinsäuretriglycerid, Triglyceridgemische mit Planzenfettsäure der Kettenlänge $C_8$-$C_{12}$ oder anderen speziell ausgewählten natürlichen Fettsäuren, Partialglyceridgemische gesättigter oder ungesättigter evtl. auch hydroxylgruppenhaltige Fettsäuren, Mono- und Diglyceride der $C_{8/10}$-Fettsäuren und andere.

Fettalkohole wie Isotridecylalkohol, 2-Octyldodecanol, Cetylstearyl-alkohol, Oleylalkohol.

Fettsäuren wie z.B. Ölsäure.

Als weitere Hilfsstoffe seien genannt:

Haftvermittler, z.B. Carboxymethylcellulose, Methylcellulose und ander Cellulose- und Stärke-Derivate, Polyacrylate, Alginate, Gelatine, Gummi-arabicum, Polyvinylpyrrolidon, Polyvinylalkohol, Copolymere aus Methylvinyläther und Maleinsäureanhydrid, Polyäthylenglykole, Paraffine, Öle, Wachse, kolloidale Kieselsäure;

Farbstoffe, die zur Anwendung am Tier zugelassen sind;

Antioxidantien wie z.B. Sulfite oder Metabisulfite wie Kaliummetabisulfit, Ascorbinsäure, Butylhydroxytoluol, Butylhydroxyanisol, Tocopherol;

Lichtschutzmittel z.B. aus der Klasse der Benzophenone oder Novantisolsäure.

Die erfindungsgemäßen Mittel lassen sich gegen alle bei Katzen parasitierenden Bandwürmern einsetzen. Dazu gehören
- Hydatigera taeniaeformis
- Dipylidium canium

3

- Joyeuxiella pasquali
- Echinococcus multilocularis

Besonders bevorzugt ist ihr Einsatz gegen Hydatigera taeniaeformis.

Als Beispiele für erfindungsgemäße Mittel seien die Mittel der folgenden Zusammensetzungen genannt:

| Beispiel 1: | |
|---|---|
| Praziquantel | 20 g |
| Isopropylmyristat | 5 g |
| Benzylalkohol | ad 100 ml |

| Beispiel 2 | |
|---|---|
| Praziquantel | 10 g |
| Isopropylmyristat | 5 g |
| Milchsäureethylester | ad 100 ml |

| Beispiel 3 | |
|---|---|
| Praziquantel | 10 g |
| Isopropylmyristat | 5 g |
| N-Methylpyrrolidon | ad 100 ml |

| Beispiel 4 | |
|---|---|
| Praziquantel | 1 g |
| Isopropylmyristat | 5 g |
| Benzylalkohol | 97,04 g |

| Beispiel 5 | |
|---|---|
| Praziquantel | 1 g |
| Isopropylmyristat | 5 g |
| N-Methylpyrrolidon | 96,19 g |

| Beispiel 6 | |
|---|---|
| Praziquantel | 1 g |
| DMSO | 30 g |
| N-Methylpyrrolidon | 73,97 g |

Beispiel A

In vivo Bandwurmtest

Taenia taeniaeformis - Katze

Experimentell mit Taenia taeniaeformis infizierte Katzen werden 6 Wochen nach Infektion einmalig durch Aufbringen von soviel Mittel, daß die angegebene Wirkstoffdosis erreicht wird, auf die Haut zwischen Hals und den Schulterblättern behandelt. Die Zahl der abgehenden Bandwürmer wird im Kot, der 0-48 h nach Behandlung abgesetzt wird, bestimmt. 6 Wochen nach der ersten Behandlung wird durch Gabe einer bekanntermaßen vollwirksamen Behandlung und anschließende Kotuntersuchung überprüft, ob Bandwürmer die erste Behandlung überlebt haben. Haben keine Bandwürmer die erste Behandlung überlebt, war die erste Behandlung voll wirksam. In der nachfolgenden Tabelle wird das Mittel sowie die zur vollen Wirksamkeit erforderliche Wirkstoffdosis in mg/kg Katze Lebendgewicht angegeben.

| Mittel gemäß Beispiel | vollwirksame Dosis Wirkstoff in mg/kg |
| --- | --- |
| 2 | 1 |
| 5 | 1 |
| 8 | 1 |
| 11 | 1 |

**Patentansprüche**

1. Verwendung von Praziquantel zur Herstellung von Mitteln zur dermalen Behandlung von Wurmerkrankungen der Katze, die Praziquantel in Konzentrationen von 0.1 - 20 Gewichteprozent neben N-Methylpyrrolidon und/oder Isopropylmyristat gegebenenfalls neben weiteren Lösungsmitteln und Hilfsstoffen enthalten und in Aufwandmengen von 0,1 - 5 mg Wirkstoff/kg Körpergewicht verabreicht werden.

2. Verwendung von Praziquantel gemäß Anspruch 1 zur dermalen Behandlung von Wurmerkrankungen der Katze in Aufwandmengen von 1 - 5 mg Wirkstoff/kg Körpergewicht.

3. Mittel zur dermalen Behandlung von Wurmerkrankungen, die Praziquantel in Konzentrationen von 0,1 - 20 Gewichtsprozent neben N-Methylpyrrolidon und/oder Isopropylmyristat enthalten.

4. Mittel zur dermalen Behandlung von Wurmerkrankungen, die Praziquantel in Konzentrationen von 0,1 - 20 Gewichtsprozent neben N-Methylpyrrolidon enthalten.

**Claims**

1. Use of praziquantel for the preparation of agents for dermal treatment of worm diseases in cats, which contain praziquantel in concentrations of 0.1-20 per cent by weight as well as N-methylpyrrolidone and/or isopropyl myristate, if appropriate, as well as further solvents and auxiliaries and are administered in application amounts of 0.1-5 mg of active compound/kg of body weight.

2. Use of praziquantel according to Claim 1 for dermal treatment of worm diseases in cats in application amounts of 1-5 mg of active compound/kg of body weight.

3. Agents for dermal treatment of worm diseases, which contain praziquantel in concentrations of 0.1-20 per cent by weight as well as N-methylpyrrolidone and/or isopropyl myristate.

4. Agents for dermal treatment of worm diseases, which contain praziquantel in concentrations of 0.1-20 per cent by weight as well as N-methylpyrrolidone.

**Revendications**

1. Utilisation du praziquantel pour la préparation de compositions destinées au traitement dermique d'helminthiases du chat, qui contiennent du praziquantel à des concentrations de 0,1 à 20 pour cent en poids à côté de N-méthylpyrrolidone et/ou de myristate d'isopropyle, le cas échéant avec d'autres solvants et substances auxiliaires, et que l'on administre en quantités de 0,1 à 5 mg de substance active par kilogramme de poids corporel.

2. Utilisation du praziquantel suivant la revendication 1 pour le traitement dermique d'helminthiases du chat en quantités de 1 à 5 mg de substance active par kg de poids corporel.

3. Compositions pour le traitement dermique d'helminthiases, qui contiennent du praziquantel à des concentrations de 0,1 à 20 pour cent en poids, à côté de N-méthylpyrrolidone et/ou de myristate d'isopropyle.

4. Compositions pour le traitement dermique d'helminthiases, qui contiennent du praziquantel à des concentrations de 0,1 à 20 pour cent en poids à côté de N-méthylpyrrolidone.